Europäisches Patentamt

European Patent Office

Office européen des brevets

(19)

(11) Veröffentlichungsnummer: **0 108 316**
**A2**

(12) # EUROPÄISCHE PATENTANMELDUNG

(21) Anmeldenummer: **83110552.3**

(22) Anmeldetag: **22.10.83**

(51) Int. Cl.³: **A 61 K 39/00**
**//(A61K39/00, 31/07, 31/59,**
**31/355, 33/04, 33/30),**
**(A61K39/00, 31/07, 31/59,**
**31/355, 33/04, 33/30, 31/425)**

(30) Priorität: **06.11.82 DE 3241113**

(43) Veröffentlichungstag der Anmeldung:
**16.05.84 Patentblatt 84/20**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Straub, Otto-Christian, Prof. Dr.**
**Im Schönblick 71**
**D-7400 Tübingen(DE)**

(54) Vakzinen mit Zuschlagstoffen.

(57) Vakzinen, die neben Antigenen fettlösliche Vitamine, physiologisch verträgliche Zinksalze und Selenverbindungen, gegebenenfalls Sorptionsvermittler und gegebenenfalls ein Spreitöl enthalten. Zusätzliche Zuschlagstoffe für die Vakzinen sind Tetramisol, Levamisol und/oder Antiprostaglandine.

Die Vakzinen dienen zur Anwendung in der Veterinärmedizin.

EP 0 108 316 A2

Croydon Printing Company Ltd.

- 1 -

BAYER AKTIENGESELLSCHAFT    5090 Leverkusen, Bayerwerk

Zentralbereich
Patente, Marken und Lizenzen    Ad/Kü-c


Vakzinen mit Zuschlagstoffen
_____


Die Erfindung betrifft Vakzinen, die neben Antigenen
fettlösliche Vitamine, physiologisch verträgliche Zink-
und Selenverbindungen, gegebenenfalls Sorptionsvermittler und gegebenenfalls ein Spreitöl enthalten.


Es wurde gefunden, daß Vakzinen, die Antigene und

a)    fettlösliche Vitamine, physiologisch verträgliche
      Zink- und Selenverbindungen sowie gegebenenfalls

b)    einen Sorptionsvermittler und gegebenenfalls

c)    ein Spreitöl enthalten,

gegenüber aus dem Stand der Technik bekannten Vakzinen
erhebliche Vorteile hinsichtlich der zellvermittelten
Immunität aufweisen.


Le A 22 036 -Ausland

Besonders bevorzugt enthalten die erfindungsgemäßen Vakzinen die anthelmintisch wirksamen Verbindungen Tetramisol oder Levamisol, d.h. ($\pm$)-2,3,5,6-Tetrahydro-6-phenyl-imidazo-/2,1-b/thiazol bzw. dessen linksdrehende Form in Mengen von 50 - 1000 mg.

Als fettlösliche Vitamine werden bevorzugt die Vitamine A, D und/oder E in den erfindungsgemäßen Vakzinen eingesetzt.

Bevorzugt enthalten die erfindungsgemäßen Vakzinen als Antigene IBR/IPV-Viren, PI-3-Viren, BVD-Viren, Adenoviren, Reoviren, RSV-Viren oder Rotaviren sowie Bakterien wie Pastorellen, Multocida und Korynebakterien in aktivierter oder inaktivierter Form.

Als besonders vorteilhaft haben sich die erfindungsgemäßen Vakzinen zur Bekämpfung und Prophylaxe von Viruserkrankungen dann erwiesen, wenn sie die oben aufgeführten Zuschlagstoffe in folgenden Mengen enthalten:

a)  20 000 bis 600 000 Einheiten fettlösliche Vitamine
A und D und
      100 bis   1 000 mg Vitamin E,
      100 bis   1 000 mg physiologisch verträgliche
Selenverbindungen und
       50 bis   1 000 mg physiologisch verträgliche
Zinkverbindungen,

b)    20    bis 95 Gewichtsteile, bevorzugt 60 bis 90 Gewichtsteile eines Sorptionsvermittlers
           und gegebenenfalls

c)    0,5  bis 50 Gewichtsteile, bevorzugt 1 bis 20 Gewichtsteile eines Spreitöls.

Bevorzugt werden 60 000 bis 600 000 Einheiten Vitamin A
und 20 000 bis 200 000 Einheiten Vitamin $D_3$ eingesetzt.

Die erfindungsgemäßen Vakzinen können als weiteren Zusatz zur Verbesserung der zellvermittelten Immunität
Rinderthymus-Extrakt enthalten. Zu diesem Zweck wird
Rinderthymus (z.B. 1 Gewichtsteil Thymus-Gewebe auf
1 Gewichtsteil RBS) homogenisiert, tiefgefroren, aufgetaut, zentrifugiert und der Überstand als Zuschlagstoff
verwendet.

Außerdem können den Vakzinen Antiprostaglandine (s. z.B.
XIIth World Congress on Diseases of Cattle, The Netherlands, World Association for Biuatrics, Proceedings Volume I,
September 7-10, 1982) in physiologisch wirksamen Mengen
zugegeben werden.

Als gut geeignete Sorptionsvermittler kommen für die
Herstellung der erfindungsgemäßen Vakzinen in Betracht:

Alkanole, wie Ethylalkohol, Isopropylalkohol, n-Butylalkohol, Amylalkohol.

Le A 22 036

Glykole, wie Propylenglykol, 1,3-Butylenglykol.

Aromatische Alkohole, wie Benzylalkohol.

Dreiwertige Alkohole, wie Glycerin.

Carbonsäureester, wie z.B. Ethylacetat, Benzylbenzoat, Butylacetat.

Aromatische und/oder aliphatische Kohlenwasserstoffe.

Öle, die nicht unter die Definition der spreitenden Öle fallen: wie z.B. Baumwollsaatöl, Erdnußöl, Maiskernöl, Olivenöl, Ricinusöl, Sesamöl.

Wasser
Ketone, wie z.B. Aceton und Methylethylketon.

Weiterhin sind u.a. Verbindungen wie Dimethylsulfoxid, Dimethylacetamid, Dimethylformamid, N-Methylpyrrolidon, Dioxan, 2-Dimethyl-4-oxymethyl-1,3-dioxalan gut als geeignet.

Besonders geeignet sind niedere Alkohole mit bis zu 7 Kohlenstoffatomen im Molekül, sowie niedere Ketone wie z.B. Aceton, Methylethylketon und niedere halogenierte Kohlenwasserstoffe wie z.B. Methylenchlorid.

Es können bei der Herstellung der erfindungsgemäßen Vakzinen ein oder mehrere Sorptonsvermittler eingesetzt werden.

Le A 22 036

Als für die Herstellung der erfindungsgemäßen Vakzinen geeignete Sorptionsvermittler kommen im Prinzip alle organischen und anorganischen Lösungsmittel in Frage, die die Antigene in ausreichender Konzentration aufnehmen und welche eine ausreichende Resorption der Vakzinen durch die Haut ohne Schädigung der Gewebe ermöglichen.

Als spreitende Öle kommen praktisch alle Substanzen in Betracht, welche die bereits oben angegebenen Eigenschaften aufweisen. Insbesondere sind die folgenden Verbindungsklassen bzw. Verbindungen geeignet:

Silikonöle verschiedener Viskosität,

Fettsäureester, wie Ethylstearat, Di-n-butyl-adipat, Laurinsäurehexylester, Dipropylen-glykolpelargonat, Ester einer verzweigten Fettsäure mittlere Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a..

Triglyceride, wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8/C_{10}$-Fettsäuren und andere.

Fettalkohole, wie Isotridecylalkohol, 2-Octyldodecanol, Cetylstearyl-alkohol, Oleylalkohol.

Fettsäuren, wie z.B. Ölsäure.

Besonders gut geeignete spreitende Öle sind die folgenden:

Isopropylmyristat, Isopropylpalmitat, Capryl/Caprinsäureester von gesättigten Fettalkoholen der Kettenlänge $C_{12}-C_{18}$, wachsartige Fettsäureester wie künstliches Entenbürzeldrüsenfett.

Als Zuschlagstoffe geeignete Zink- und Selenverbindungen sind:

Zinkhalogenide wie Zinkjodid, Zinkfluorid, Zinksulfat, Zinkmyristat, Zinkdinatriumsalz der Ethylendiamintetraessigsäure, Zinkphosphate, Zinktartrate, Zinkcitrate in physiologisch verträglichen Mengen.

Selenhalogenide wie Selenchlorid, Selenfluorid;

Selenide wie $Na_2Se$; Selenoxid; selenige Säure, Selenite etc. in physiologisch verträglichen Mengen.

Die Vakzinen werden parenteral durch Impfung oder Aufsprühen lokal appliziert (z.B. zur Bekämpfung von IBR/IPS im Atmungs-, Verdauungs- oder Genitaltrakt von Rindern).

Zur Herstellung der Vakzinen werden die Antigene und die Zusätze gemischt und homogenisiert. Der pH-Wert wird mittels geeigneter Puffer auf 5 bis 8, bevorzugt jedoch neutral eingestellt. Wasser und andere pharmakologisch unbedenkliche Lösungsmittel dienen als Träger für die erfindungsgemäßen Vakzinen.

Die Anwendungsdosis der erfindungsgemäßen Vakzinen beträgt in der Regel 2 bis 10 ml pro Tier.

Versuchsbericht 1 / Rinder

2 Gruppen von Rindern (19 Tiere / 25 Tiere) wurden mit inaktivierter IBR/IPV Virussuspension (inaktiviert mit Ethylenimin), die einmal die erfindungsgemäßen Zuschlagstoffe enthielt (19 Tiere) und zum anderen ohne Zuschläge (25 Tiere) verwendet wurde, subkutan geimpft. Nach 6 Wochen wurde die Impfung wiederholt und nach 9 Monaten der Titer im Serumneutralisationstest bestimmt. Die Tiere, die mit den erfindungsgemäßen Zuschlägen geimpft worden waren, wiesen im Durchschnitt einen Titer von 1:10,4 auf. Ohne Zuschläge betrug der Titer 1:6.

Der pH-Wert des Impfstoffes betrug 5,2. Als Dosis wurden 2,5 ml eingesetzt.

Le A 22 036

- 8 -

Zuschlagstoffe

0,5 ml handelsübliche Vitamin A, D und E
Präparation entsprechend
150 000 Einheiten A
100 000 Einheiten D und
150 mg E

0,5 ml 10 % Levamisol-Lösung;
0,25 ml Zn-Salzlösung ensprechend    50 mg Zn
0,25 ml Na-Selenit-Lösung entsprechend 100 mg Se;

1,25 g Medium (Wasser).

Irritationen der Haut bzw. Temperaturerhöhungen konnten
nach Applizierung dieser Vakzine nicht beobachtet werden.

Versuchsbericht 2 / Schafe

Es wurde ein Vakzinierungsversuch an Schafen durchgeführt. Dazu wurden die Tiere im Abstand von 2 Monaten
mit einer inaktivierten IBR/IPV-Vakzine (inaktiviert
mit Ethylenimin).

a)    ohne Zuschläge und

b)    mit den Zuschlagstoffen gemäß Versuchsbericht 1
geimpft.

Le A 22 036

Der pH-Wert des Impfstoffes mit Zuschlägen betrug 5,2.

Der Serumneutralisationstest ergab folgende Titer in
-log 10.

| 2 Monate nach der 1. Impfung | mit a) | 0,45 |
|---|---|---|
| | und | 0,00 |
| | mit b) | 1,05 |
| | und | 0,90 |
| 1 Monat nach der 2. Impfung | mit a) | 1,20 |
| | und | 0,75 |
| | mit b) | 1,95 |
| | und | 1,35. |

Demnach bewirken die Zuschläge eine erhebliche Verbesserung der immunogenen Wirkung der Vakzinen.

Le A 22 036

Patentansprüche

1. Vakzine enthaltend Antigene und

   a) fettlösliche Vitamine und physiologisch verträgliche Zink- und Selenverbindungen sowie gegebenenfalls

   b) einen Sorptionsvermittler und gegebenenfalls

   c) ein Spreitöl.

2. Vakzine nach Anspruch 1, dadurch gekennzeichnet,
   daß sie Levamisol und/oder ein Antiprostaglandin
   enthält.

3. Vakzine nach den Ansprüchen 1 und 2, dadurch gekennzeichnet, daß sie die Vitamine A, D und/oder
   E enthält.

4. Vakzine nach Anspruch 1 enthaltend

   a)   20 000 bis 600 000 Einheiten fettlösliche
                         Vitamine A und D und
        100 bis   1 000 mg Vitamin E,
        100 bis   1 000 mg phsiologisch verträgliche
                         Selenverbindungen und
         50 bis   1 000 mg physiologisch verträgliche
                         Zinkverbindungen,

Le A 22 036

b)     20   bis   95 Gewichtsteile eines Sorptionsvermittlers und gegebenenfalls

c)    0,5 bis   50 Gewichtsteile eines Spreitöls.

5. Vakzine nach Anspruch 4 enthaltend 50 bis 1000 mg
Levamisol.

6. Vakzine nach Anspruch 1, dadurch gekennzeichnet,
daß sie als Sorptionsvermittler eine Verbindung aus
der Gruppe bestehend aus Alkanolen, Glykolen, aromatischen Alkoholen, dreiwertigen Alkoholen, Carbonsäureestern, aromatischen und aliphatischen
Kohlenwasstoffen, nicht-spreitenden Ölen, Wasser
und Ketonen enthält.

7. Vakzine nach Anspruch 1, dadurch gekennzeichnet,
daß sie Dimethylsulfoxid oder Isopropylalkohol
und gegebenenfalls Isopropylmyristat als Spreitöl
enthält.

8. Vakzine nach den Ansprüchen 1 bis 7, dadurch gekennzeichnet, daß sie als Antigene IBR/IPS-Viren, PI-
3-Viren, BVD-Viren, Adenoviren, Reoviren, RSV-
Viren, Retroviren, Enteroviren, Corona-Viren,
Pocken-Viren oder Rotaviren sowie Bakterien wie
Pastorellen, Multocita und Koryne-Bakterien in
aktivierter oder inaktivierter Form enthält.

- 12 -

9. Verwendung von fettlöslichen Vitaminen, physiologisch verträglichen Selen- und Zinkverbindungen und Sorptionsvermittlern, gegebenenfalls in Verbindung mit Levamisol und/oder Antiprostaglandinen und gegebenenfalls in Verbindung mit Spreitölen als Zuschlagstoffe zu Antigene enthaltenden Vakzinen.

10. Verwendung von Dimethylsulfoxid als Sorptionsvermittler in Antigene enthaltenden Vakzinen.

Le A 22 036